# EUROPEAN PATENT SPECIFICATION

(11) **EP 3 943 110 B1**
(45) Date of publication and mention of the grant of the patent: **18.10.2023**
(21) Application number: 20187269.4
(22) Date of filing: 22.07.2020
(51) Int. Cl.: A61K 41/00, A61P 31/12, A61P 31/14

(54) **USE OF PHOTOSENSITIZERS FOR TREATMENT OF VIRAL RESPIRATORY INFECTIONS**
VERWENDUNG VON PHOTOSENSIBILISATOREN ZUR BEHANDLUNG VON VIRALEN ATEMWEGSINFEKTIONEN
UTILISATION DE PHOTOSENSIBILISATEURS POUR LE TRAITEMENT DES INFECTIONS VIRALES RESPIRATOIRES

(43) Date of publication of application: 26.01.2022
(73) Proprietor: Arentz, Jochen, 22301 Hamburg (DE); Albrecht, Hans, 81479 München (DE)
(72) Inventor: Arentz, Jochen, 22301 Hamburg (DE)
(74) Representative: Habermann, Hruschka & Schnabel

(56) References cited:
- CN-A- 111 346 108
- MARKUS EICKMANN ET AL: "Inactivation of three emerging viruses - severe acute respiratory syndrome coronavirus, Crimean-Congo haemorrhagic fever virus and Nipah virus - in platelet concentrates by ultraviolet C light and in plasma by methylene blue plus visible light", VOX SANGUINIS., vol. 115, no. 3, 12 January 2020 (2020-01-12), pages 146-151, XP055737528, CH ISSN: 0042-9007, DOI: 10.1111/vox.12888
- MARKUS EICKMANN ET AL: "Inactivation of Ebola virus and Middle East respiratory syndrome coronavirus in platelet concentrates and plasma by ultraviolet C light and methylene blue plus visible light, respectively : EBOV AND MERS-CoV INACTIVATION", TRANSFUSION, vol. 58, no. 9, 6 May 2018 (2018-05-06), pages 2202-2207, XP055762794, US ISSN: 0041-1132, DOI: 10.1111/trf.14652
- Jin Changzhong: "Methylene blue photochemical treatment as a reliable SARS-CoV-2 plasma virus inactivation method for blood safety and convalescent plasma therapy for the COVID-19 outbreak", , 17 March 2020 (2020-03-17), pages 1-15, XP055737518, Retrieved from the Internet: URL:https://www.researchsquare.com/article /rs-17718/v1.pdf? [retrieved on 2020-10-07]
- Abeer Tawfik: "Photodynamic Therapy for the Treatment of COVID-19", , 4 June 2020 (2020-06-04), XP055762647, Retrieved from the Internet: URL:https://clinicaltrials.gov/ct2/show/re cord/NCT04416113 [retrieved on 2021-01-04]
- MOGHISSI K ET AL: "Does PDT have potential in the treatment of COVID 19 patients?", PHOTODIAGNOSIS AND PHOTODYNAMIC THERAPY, ELSEVIER, AMSTERDAM, NL, vol. 31, 24 June 2020 (2020-06-24), XP086273406, ISSN: 1572-1000, DOI: 10.1016/J.PDPDT.2020.101889 [retrieved on 2020-06-24]
- ADELAIDE ALMEIDA ET AL: "Antimicrobial Photodynamic Therapy in the Control of COVID-19", ANTIBIOTICS, vol. 9, no. 6, 11 June 2020 (2020-06-11), page 320, XP055762797, DOI: 10.3390/antibiotics9060320
- Deepak Golwalkar: "Treatment for COVID-19 using Methylene Blue | by Dr Deepak Golwalkar | Medium", , 13 April 2020 (2020-04-13), pages 1-6, XP055737620, Retrieved from the Internet: URL:https://medium.com/@dr.deepak.golwalka r/treatment-for-covid-19-using-methylene-b lue-d23fc5a31a4d [retrieved on 2020-10-07]
- CORONA M CASSIDY ET AL: "Drug and light delivery strategies for photodynamic antimicrobial chemotherapy (PACT) of pulmonary pathogens: A pilot study", PHOTODIAGNOSIS AND PHOTODYNAMIC THERAPY, vol. 8, no. 1, 1 March 2011 (2011-03-01), pages 1-6, XP028145244, ISSN: 1572-1000, DOI: 10.1016/J.PDPDT.2010.12.007 [retrieved on 2010-12-23]
- GERALDE MARIANA C ET AL: "PDI using nebulized indocyanine green for pneumonia treatment", PROGRESS IN BIOMEDICAL OPTICS AND IMAGING, SPIE - INTERNATIONAL SOCIETY FOR OPTICAL ENGINEERING, BELLINGHAM, WA, US, vol. 10470, 14 February 2018 (2018-02-14), pages 104700H-104700H, XP060101820, ISSN: 1605-7422, DOI: 10.1117/12.2291987 ISBN: 978-1-5106-0027-0
- GIULIA KASSAB ET AL: "Nebulization as a tool for photosensitizer delivery to the respiratory tract", JOURNAL OF BIOPHOTONICS, vol. 12, no. 4, 26 December 2018 (2018-12-26), XP055762928, DE ISSN: 1864-063X, DOI: 10.1002/jbio.201800189
- DIAS LUCAS D ET AL: "COVID-19: Beyond the virus. The use of photodynamic therapy for the treatment of infections in the respiratory tract", PHOTODIAGNOSIS AND PHOTODYNAMIC THERAPY, ELSEVIER, AMSTERDAM, NL, vol. 31, 8 May 2020 (2020-05-08), XP086273337, ISSN: 1572-1000, DOI: 10.1016/J.PDPDT.2020.101804 [retrieved on 2020-05-08]
- MARIANA C. GERALDE ET AL: "Pneumonia treatment by photodynamic therapy with extracorporeal illumination - an experimental model", PHYSIOLOGICAL REPORTS, vol. 5, no. 5, 1 March 2017 (2017-03-01), page e13190, XP055762924, US ISSN: 2051-817X, DOI: 10.14814/phy2.13190
- NOGUEIRA MARCELO SAITO: "Optical theranostics and treatment dosimetry for COVID-19 lung complications: Towards increasing the survival rate of vulnerable populations", PHOTODIAGNOSIS AND PHOTODYNAMIC THERAPY, ELSEVIER, AMSTERDAM, NL, vol. 31, 22 June 2020 (2020-06-22), XP086273409, ISSN: 1572-1000, DOI: 10.1016/J.PDPDT.2020.101892 [retrieved on 2020-06-22]
- Anonymous: "Steriwave - New Technology Reduces Healthcare-Associated Infections and Surgical Site Infections Steriwave Nasal Decolonization (ND) System", , 8 May 2020 (2020-05-08), XP093014190, Retrieved from the Internet: URL:https://ondinebio.com/wp-content/uploa ds/2019/03/SW9001-Rev-A-Steriwave-brochure .pdf [retrieved on 2023-01-16]
- SINGANAYAGAM A: "Duration of infectiousness and correlation with RT-PCR cycle threshold values in cases of COVID-19, England, January to May 2020", EURO SURVEILL, vol. 25, no. 32, 13 August 2020 (2020-08-13), pages 1-5, XP093061847,

## Description

The present invention relates to photosensitizers for use in the prevention and/or treatment of viral respiratory infections caused by a coronavirus as defined in claim 1. The present invention also relates to a photosensitizer for use as defined in claim 14 wherein the photosensitizer is comprised in a pharmaceutical device, the pharmaceutical device comprising a nebulizer so as to administer the photosensitizer into at least a part of the respiratory tract of a subject to be treated. As defined in claim 17, the invention furthermore relates to medical kits comprising a device of the invention and a handheld light source.

Photosensitizers have been used for photodynamic therapy (PDT) of infections by bacteria and fungi as well as cancer and tumours. Photosensitizers are generally dyes which are transferred to a higher energetically state by absorption of light typically in the visible wavelength range. In the photo-activated state the photosensitizer is able to transfer, as a donor, at least part of the energy to an acceptor molecule. For application in PDT, the energy transfer leads, at least in part, to reactive oxygen species (ROS) such as the superoxide radical which in turn damages various cellular components, especially the cell membrane, of the target cells such as tumour cells, bacteria, or fungal cells, by redox reactions. PDT is mostly applied through local application of the dye and light typically emitted by monochromatic and/or coherent light sources.

The article "Does PDT have potential in the treatment of COVID 19 patients?" (24 June 2020, Photodiagnosis and Photodynamic therapy, vol. 31) by K. Moghissi describes a methodology based on Methylene Blue mediated PDT used topically within the airway and suggests that this methodology could be modulated for use in respiratory infections of COVID -19.

The article "Antimicrobial Photodynamic Therapy in the Control of COVID-19" (11 June 2020, Antibiotics, vol. 9, no. 6) by Adelaide Almeida et al describes aPDT which might help to mitigate COVID-19.

"Treatment for COVID-19 using Methylene Blue" (13 April 2020, URL:https://medium.com/@dr.deepak.golwalkar/treatment-for-covid-19-using-methylene-blue-d23fc5a31 a4d) by Deepak Golwalkar describes using methylene blue in nebulized form for treating patients with coronavirus (covid-19). The article "Methylene blue photochemical treatment as a reliable SARAS-CoV-2 plasma virus inactivation method for blood safety and convalescent plasma therapy for the COVID-19 outbreak" (17 March 2020, pages 1 to 15; available at https://researchsquare.com/article/rs-17718/v1.pdf) by Jin Changzhong describes the inactivation of SARS-CoV-2 in human plasma by using methylene blue and subsequent irradiation of the plasma.

The technical problem underlying the present invention is to provide improved treatment of coronaviral infections in the respiratory tract as well as novel therapeutic devices and kits for performing the treatment.

In particular, the present invention provides, under a first aspect, a photosensitizer for use in the treatment of viral respiratory infections caused by a coronavirus as defined in claim 1.

In order to provide the photosensitizer at the required amount and concentration at the region of the targeted viruses, the photosensitizer is applied inra-nasally and/or intra-orally and/or intra-pharyngeally.

As mentioned above, the photosensitizer needs to be activated in order to exert its function of at least damaging or, optimally, eliminating coronaviruses infecting the respiratory tract of the subject to be treated, which is accomplished by light emission on the part or region of the subject in need of the inventive treatment where the photosensitizer has been applied to. The light emission is directed to the respiratory tract, or at least a part thereof, of the subject, wherein the light emission is carried out by irradiating at least a part of the respiratory tract from the outside of the subject. The light for irradiating at least a part of the respiratory tract has a wavelength, or at least comprises a wavelength, selected from 550 nm to 820 nm of the electromagnetic spectrum, and typically has also an intensity, preferably in terms of energy density, i.e. applied energy per surface area, causing the photosensitizer to be transferred to an active state as outlined above.

According to the invention the treatment comprises irradiating at least a part of the respiratory tract of a subject from outside of the subject with light comprising or having a wavelength as outlined above causing the photosensitizer to be transferred to an active state. It is conceived that one or more different parts of the respiratory tract may be irradiated one or more times for a time effective to provide the sufficient light energy so as to effect the inventive transfer of the photosensitizer, in particular the effective amount administered to the respiratory tract, or one or more parts thereof, to the active state at least in the irradiated part or region, or whole of, respectively, of the respiratory tract. Parts of the respiratory tract which may be conveniently irradiated from the outside of the subjects body include, e.g. nose, mouth (preferably, the subject's mouth can be irradiated while in the opened or at least partially opened state), cheeks (one side or both sides, preferably left and right cheek simultaneously or sequentially), neck, throat, chest and/or back of the subject to be treated. It is to be understood that the term "from the outside of the subject's body" means that the irradiation is directed to the skin of the subject in the respective area, in the following also denoted as "transcutaneous irradiation", with two exceptions, namely, the mouth and the nose: as outlined before, the mouth of the subject can be irradiated in an open state where the emitted light will also, or mainly, irradiate the oro-pharyngeal part of the respiratory tract of the treated subject. The nose of the subject can also be treated by emitting light into the nostrils of the patient so that the light also, or mainly, reaches the nasal part of the subject's respiratory tract. In any case, however, irradiation from the outside of the subject's body means that the light-emitting device is not introduced into any parts of the respiratory tract.

The light source used for irradiation according to the invention, which, according to the invention, may refer to light emitting element of a corresponding device or the device *per se*, is preferably a device comprising a light emitting diode (LED). For convenience of use, the LED device is preferably a handheld light emitting device, more preferably a handheld LED device such as a device having the form and comprising the typical parts of, respectively, a torch or flashlight, respectively. In embodiments of the invention the light source emits coherent light, in which case the light source is a laser device emitting light of a specific wavelength. The laser device can be an LED laser device, and in certain embodiments, it may be a handheld laser LED device, as outlined above. In other preferred embodiments, the light source or light emitting device, respectively, can be a light source or device, respectively, emitting non-coherent light, preferably an LED device, more preferably a handheld LED device. It is a surprising finding according to the invention that it is sufficient for attaining substantial and strong virus inhibition to use a broadband light source, more preferably a broadband LED device, more preferably a broadband handheld LED device. In preferred embodiments of broadband LED devices, the LED light source is doted with Cer which shifts the potential dominant blue part (shorter wavelength) of the emitted light to longer wavelengths. In other preferred embodiments the LED devices can be one emitting non-coherent monochromatic light having a wavelength as defined in claim 1.

The photosensitizers for use in the invention have a structure according to the following general formula (I): 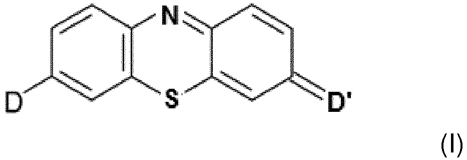 with D and D' each being an electron donor group, and wherein the positions C-1, C-2, C-4, C-6, C-8 and/or C-9 of the phenothiazine ring system may be substituted by a substituent preferably selected from substituted or unsubstituted lower alkyl, preferably C₁₋₃-alkyl, particularly preferred methyl, or lower alkenyl, preferably C₂₋₃-alkenyl, amino, halo, and cyano. Preferably, D and D' are each independently a NRR' group with R and R' being preferably independently selected from H, substituted or unsubstituted lower alkyl, preferably C₁₋₃-alkyl, particularly preferred methyl, and lower alkenyl, preferably C₂₋₃-alkenyl.

Particularly preferred phenothiazine derivatives for use in the invention are methylene blue, new methylene blue, toluidine blue and mixtures thereof.

The photosensitizer for use in the invention is typically applied in form of a composition containing at least one photosensitizer according to formula (I) in combination with at least one excipient and/or diluent, preferably at least one pharmaceutically acceptable excipient and/or diluent. Preferred diluents/excipients for use in the invention are typically water, preferably sterilized water, more preferably sterilized distilled water, or aqueous solutions such saline or buffered saline solutions such as PBS, whereby it is understood that such diluents and/or excipients are preferably sterilized.

It has been surprisingly found according to the invention that photosensitizers of above formula (I), particularly preferred methylene blue, new methylene blue, toluidine blue or mixtures thereof, can be applied in comparatively very low concentrations for effectively combating viruses causing respiratory conditions. In preferred embodiments the composition comprises a photosensitizer for use in the invention, particularly preferred a phenothiazine derivative as defined herein, in a concentration of from about 0.00001 % (w/v) to about 0.1 % (w/v), preferably from about 0,0001 % (w/v) to about 0,001 % (w/v).

The photosensitizer, preferably contained in a composition as outlined above, is administered to the respiratory tract of the subject through intra-nasal and/or intraoral and/or intra-pharygeal application. For example, the photosensitizer, in particular the composition containing at least one photosensitizer (i.e. one or more of such photosensitizers) as outlined above may be administered via a pipette or similar device. For administration to deeper parts of the respiratory tract tube systems known in the art equipped with a syringe reservoir at its proximal part can be used. In preferred embodiments, allowing for reaching the upper and lower parts of the respiratory tract of the subject to be treated, the photosensitizer(s) is (are) preferably administered by use a device configured to administer the photosensitizer(s), preferably a composition containing the photosensitizer(s), in spray or nebulized from. Typically, such a device comprises a nebulizer element and a container comprising a composition containing one or more photosensitizers as described above.

The invention is also directed to photosensitizers for use as defined in claim 14 wherein the photosensitizer is comprised in such medical devices for administration of the photosensitizers in spray or nebulized, respectively, form. In particular, the present invention provides a photosensitizer for use according to claim 14, wherein the photosensitizer is comprised in a pharmaceutical device, the pharmaceutical device comprising a nebulizer and a container containing at least one photosensitizer of general formula (I), optionally in combination with at least one pharmaceutically acceptable excipient and/or diluent, wherein preferred embodiments have been already described in detail herein-above.

As already noted above, the wavelength or wavelength spectrum of the light emitted by the light source depends on the particular photosensitizer of formula (I). Preferred phenothiazine photosensitizers for use in the invention typically absorb light in the visible to near infra red light of the electromagnetic wave spectrum. For example, in the visible spectrum, methylene blue absorbs light in the wavelength region of from about 530 nm to about 700 nm, new methylene absorbs light in the wavelength region of from about 500 nm to about 700 nm, and toluidine blue absorbs light in the region of from about 520 nm to about 700 nm. According to the invention, the photosensitizer is activated by irradiation with light having a wavelength or comprising wavelengths, respectively, in the range of from about 500 nm to about 820 nm. As already indicated above, the light source or light emitting device, respectively, for use in the invention emits light within this wavelength region, whereby the light source can emit light in broadband region, preferably, of the above indicated visible to near infrared spectrum, preferably of about 500 nm to about 820 nm. Particularly preferred, the wavelength used for activating the photosensitizer, preferably one or more of the above-described phenothiazines, preferably, methylene blue and/or new methylene blue and/or toluidine blue, most preferred methylene blue, is at or around 590 nm such as from about 550 nm to about 630 nm, preferably about 570 to about 610 nm, more preferably about 585 nm to about 595 nm, most preferred 590 nm. Preferably, such light is emitted by an LED device, preferably a handheld device, which can in certain preferred embodiments of the invention emit non-coherent light.

In other preferred embodiments of the invention, the invention makes use of the light absorption of phenothiazine photosensitizers according to formula (I) in the near infrared region as outlined above. In certain embodiments of this type, the photosensitizer-activating light is in the near infrared region of the electromagnetic spectrum, preferably from about 780 nm to about 820 nm, more preferably around or at 810 nm such as from about 800 nm to about 820 nm, most preferred 810 nm. Near infrared light has the advantage that it is absorbed to a lesser extend by body components so that it can reach into deeper into the irradiated regions as defined above. The fact of the wavelength dependency of light scattering which is higher for longer wavelengths further contributes to this effect, and at wavelengths of at or around 810 nm the scattering coefficient is higher than the absorption coefficient, and furthermore the scattering is a forward scattering further contributing to the higher depth of penetration. Such light preferably has a depth of penetration of about 5 to 10 cm which makes it particularly suitable for irradiation from the outside of the body.

In the context of irradiation in the near infrared region of the electromagnetic spectrum, preferably from about 780 nm to about 820 nm, more preferably around or at 810 nm such as from about 800 nm to about 820 nm, most preferred about 810 nm, the present invention also provides a the use of a photosensitizer of general formula (I) for photodynamic treatment, namely for treatment of infections by a coronavirus of the respiratory tract, more preferably for treatment and/or prevention of the coronaviral infections as described above, by application of a photosensitizer according to the definition as outlined above, more preferably selected from methylene blue, new methylene blue and toluidine blue, to a desired region, especially to a region of the respiratory tract, namely, intra-nasally and/or intra-orally and/or intra-pharyngeally, of the subject to be treated, preferably a human, and transcutaneous irradiation of said region.

Depending on the particular photosensitizer selected from those of general formula (I), it may be desired for efficient energy transfer to select a wavelength or wavelength region, respectively, at or at least near the absorption maximum of the photosensitizer. "Near the absorption maximum" in the context of the invention typically means a range around the absorption maximum of from about - 50 nm to about + 50 nm, preferably from about -20 nm to about + 20 nm, more preferably from about -10 nm to about + 10 nm of the absorption maximum. The absorption characteristics, including an absorption maximum of the photosensitizer, preferably of a phenothiazine derivative as described herein, is typically in the visible to near infrared range of the light spectrum, will depend on the biological setting, i.e. in which the photosensitizer is present as well as whether the subject is irradiated from outside of the body or within the body, and will also depend on and thus can be tuned by the components of a composition in which the photosensitizer(s) is present for application.

In other embodiments of the invention, the wavelength used for activating the photosensitizer is selected from regions of the, typically visible, absorption spectrum being more remote from an absorption maximum of the respective photosensitizer. In this context of the invention, the term "remote from an absorption maximum" of a photosensitizer for use in the invention refers to a wavelength.

The duration and number of times of irradiation of the at least part of the respiratory tract of the subject to be treated according to the invention varies according the specific photosensitizer and the wavelength or wavelength region of the light. Typically, the irradiation is carried out from the outside of the subject's body one or more times, such as 1, 2, 3, 4, 5, 6, 7, 8, 9, 10 times or more in one or more sessions at a day of treatment. The treatment can be repeated for two or more days, wherein the days of treatment can be uninterrupted or interrupted by one or more days where no treatment takes place. Preferably, the duration of the irradiation per irradiation incidence (i.e. one or more times as outlined above) is from about 1 min to about 20 min, more preferably from about 2 to about 20 min, particularly preferred from about 3 to about 8 min.

In preferred embodiments of the invention the at least part of the respiratory tract is irradiated from outside of the subject with an energy density of 50 to 150 J/cm², preferably 80 to 120 J/cm,^{,} more preferably 90 to 110 J/cm, most preferably 100 J/cm². Preferred emitted light intensities, especially in the case of irradiation with non-coherent light, preferably by LED devices, more preferably handheld LED devices, such as broadband LED devices or LED devices emitting light at about or around about 590 nm (as outlined above) range from about 6000 Lux to about 90000 Lux, preferably from about 10000 Lux to about 70000 Lux, most preferred from about 40000 Lux to about 60000 Lux, such as, particularly preferred about 50000 Lux.

The viruses targeted by the invention are coronaviruses causing respiratory infections, more preferably coronaviruses causing severe respiratory conditions. Preferably, the photosensitizer for use in the inventive treatment according to claim 1 is directed against SARS-CoV, MERS-CoV, SARS-CoV 2, HCoV-HKU1, HCoV-OC43, HCoV-NL63, HCoV-229E and/or bovine coronaviruses (bCoVs), with SARS-CoV-2 being the most preferred targeted virus which causes the COVID-19 disease.

In particular as regards coronaviruses such as those as outlined above, especially SARS-CoV-2, the invention is preferably carried out at an early stage of the infection. In such preferred embodiments, the term "early stage of the infection" means that the infecting coronavirus has infected the subject, preferably a human subject, such that it has entered the respiratory tract, in particular the upper parts of the respiratory tract such as mouth, nose and/or pharyngeal part, but has not reached or has not reached in substantial extent, respectively, the lower parts of the respiratory tract, in particular the bronchia. In this context, especially with respect to the coronaviruses SARS-Co-V, MERS and, particularly preferred, SARS-CoV-2, the viral infection has not resulted in a viral pneumonia. Thus, the photosensitizer for use in the inventive treatment according to claim 1 is preferably used for preventing a viral pneumonia due to infection by a coronavirus, preferably due to an infection by SARS-CoV, MERS, or SARS-CoV-2. According to the invention as defined in claim 1, the photosensitizer for use in the inventive treatment can also be used for prevention and/or treatment of a coronaviral respiratory infection by applying the invention to a subject which is suspected to be infected by a coronavirus causing a respiratory infection or disease state, respectively. For example, especially in the case of coronaviruses causing severe respiratory conditions such as bronchitis and/or pneumonia, such as SARS-CoV, MERS and, particularly preferred in the context of the invention, SARS-CoV-2, the subject can be treated at a time point after, preferably shortly after such as about 15 min, about 30 min, about 1 h, about 2 h, about 3 h, about 4 h, about 5 h, about 6 h, about 7 h, about 8 h, about 9 h, about 10 h, about 11 h, about 12 h or about 24 hours, the subject has gained information and/or has become aware and/or has been made aware of that the subject had close contact such as contact within a distance of below about 3 m, preferably below about 2 m, more preferably below about 1.5 m, and typically for a sufficient amount of time such as at least about 30 s, preferably at least about 1 min, more preferably at least about 2 min, with a subject having a coronaviral respiratory infection such as an infection by SARS-CoV, MERS and/or, particularly preferred, SARS-CoV-2.

The present invention is also directed to a medical kit as defined in clam 17 comprising a pharmaceutical device as described and defined herein and a handheld light source emitting light comprising or having a wavelength causing the photosensitizer to be transferred to an active state. Preferred embodiments of such light sources have been elaborated above.

The present invention is directed to a photosensitizer for use in the prevention and/or treatment of a respiratory infection caused by coronaviruses as defined in claim 1, preferably those as outlined herein before, comprising applying the photosensitizer to at least a part of the respiratory tract of the subject, namely intra-nasally and/or intra-orally and/or intra-pharyngeally and irradiating said at least part of the respiratory tract of the subject from the outside with light having a wavelength causing the photosensitizer of formula (I) to be transferred in to an activated state, namely with light comprising or having a wavelength selected from 500 nm to 820 nm of the electromagnetic spectrum.

Preferred embodiments of photosensitizers and compositions containing same, administration regimens, light sources and light emitting devices, wavelengths, duration and times of irradiation for use in the invention have already described herein above.

Further preferred details and embodiments the invention, in particular treatment conditions as well as devices, photosensitizers, light sources etc. have been described herein-before and will further be exemplified in the examples.

The Figures show:
Fig. 1 shows a table illustrating the irradiation conditions used in Example 1.
Fig. 2 shows a table illustrating the results of the experiments according to Example1.
Fig. 3 shows a table illustrating the results of the experiments according to Example 2 (VC: virus control; MV: mean value; SD: standard deviation; RF: reduction factor; n.a.: not applicable; n.d.: not done; the results of the experiments according to the last two lines of the table were obtained after immediate wrapping of the experimental plate with aluminium foil and storage in the dark (before freezing).

The present invention is further illustrated by the following non-limiting examples:

### EXAMPLES

### Example 1: Inactivation of bovine coronavirus in infected cells in vitro

### Materials

### Laser device

Photolase 810 Diode Laser of the applicant: max. Laser Output Power: 7 Watt; Laser Wavelength: 810 nm/7 Watt)

The laser device contains a handpiece allowing irradiation without loss of performance in a distance up to 105 cm with a light cone laser of approximately 1 cm diameter. In some experiments, the laser was fixed on a tripod in a distance of 30 cm to ensure the same conditions for all tests.

### Irradiation conditions

Power density: 0.3 Watt
Irradiation Intensity: 100 Joule/cm²
Amount of sensitizer/well of 48-well plate: 0.1 %
0.01 %
0.001 %
0.0001 %
Test temperature (irradiation): 20°C ± 2°C
Incubation temperature (titration): 37°C ± 1°C

### Photosensitizer

Photolase Blueviolett Sensitizer 810: Methylene blue stock solution containing 1.07 % (w/v) methylene blue.

### Cells and viruses

BCoV strain L9 was obtained from Dr. G. Zimmer, Institute of Virology at the Tierärztliche Hochschule Hannover, Germany.

U373 cells (passage 14) were obtained from Dr. G. Zimmer, Institute of Virology at the Tierärztliche Hochschule Hannover, Germany.

### Media and auxiliary reagents

Eagle's Minimum Essential Medium with Earle's BSS (EMEM, Biozym Scientific GmbH, catalogue no. 880121)
Fetal calf serum (FCS, Sigma-Aldrich, article no. F 7524)
Aqua bidest. (SG ultrapure water system, type Ultra Clear; serial no. 86996-1)
PBS (Invitrogen, article no. 18912-014)
Trypsin-EDTA solution
Penicillin/ streptomycin (Sigma-Aldrich, article no. P-0781)
Trypsin stock solution [2.5 mg/ml]

### Methods

To analyse the efficacy of the inventinve photoactivated treatement for inactivation of bovine coronavirus, U373 cells were cultivated in 48-well plates and infected with BCoV before irradiation treatment. The following general steps were carried out:
(1) (Sub)culturing of U373 cells in 48-well plates
(2) 150 µl cell suspension + 500 µl medium (EMEM 10 % FKS)
(3) Infection of the U373 cells with 200 µl of virus-medium-mixture
(4) Pre-treatment and irradiation of the BCoV-infected cells

### Preparation of test virus suspension

For preparation of test virus suspension, U373 cells were cultivated in a 175 cm2 flask with in EMEM supplemented with L-glutamine,non-essential amino acids and sodium pyruvate and 10 % fetal calf serum. Before virus infection, cells were washed two times with phosphate buffered saline (PBS), incubated for 3 h with EMEM without FCS whereafter the cells were washed once with EMEM supplemented with trypsin. For virus production, BCoV strain L9 was added to the prepared monolayer. After an incubation period of 24 to 48 hours (cells showed a constant cytopathic effect), cells were lysed by a rapid freeze/thaw cycle.

Cellular debris was removed by low speed centrifugation. After aliquotation of the supernatant, test virus suspension was stored at -80 °C.

### Preparation of U373 cells for irradiation treatment

U373 cells of a 175 cm2 cell culture flask were detached enzymatically with Trypsin-EDTA solution and taken up in a total of 60 ml of EMEM medium with 10 % fetal calf serum. 150 µl each of this cell suspension were transferred into six wells of a 48-well plate with a final volume of 650 µl by adding 500 µl EMEM/10 % FCS.

48-well plates were selected, since the diameter of one well corresponds to the diameter of the laser cone (light cone laser: approx. 1 cm diameter; well of 48-well plate: 1.04 cm diameter), which ensures that the cells are treated evenly throughout the entire well during radiation.

After three days of cultivation at 37 °C and 5 % CO2, cells were washed two times with EMEM without FCS (2 x 200 µl per well) and incubated for further 3 h at 37 °C and 5 % CO2. For virus infection, medium was removed from the individual wells and replaced by 200 µl virus-medium-mixture (500 µl BCoV virus suspension were mixed with 30 ml EMEM without FCS/ with penicillin/streptomycin, and trypsin). After an incubation period of 20 to 24 hours BCoV-infected cells were used for irradiation treatment.

### Preparation of photosensitizer

The methylene blue photosensitzier Photolase^{®} Blueviolett Sensitizer 810 was used in the following concentrations based on the content of 1.07 % (w/v) methylene blue in the undiluted sensitizer:
(a) 0.1 % (w/v) (end concentration in 200 µl /well)
(b) 0.01 % (w/v) (end concentration in 200 µl /well)
(c) 0.001 % (w/v) (end concentration in 200 µl /well)
(d) 0.0001% (w/v) (end concentration in 200 µl /well)

### Irradiation procedure

For photodynamic inactivation of the bovine coronavirus (BCoV) first the undiluted or appropriately diluted (see above) photosensitizer solution (18.69 µl per well) was added to the BCoV-infected cells for 1 minute. Thereafter, irradiation with the above laser device was performed with an intensity of 100 joule/cm² for 5.46 min under constant power of 0.3 watt/cm2 and artificial laboratory light.

After treatment, plates were stored at -80 °C, successively.

### Recovery of the residual virus and determination of infectivity

For recovery of residual virus from the infected and treated cells, plates were subjected to a rapid freeze/thawing procedure. This was followed by mixing of cell suspension in each well by pipetting up and down 10 times to re-suspend the virus. Then, series of ten-fold dilutions of the suspensions were prepared in ice-cold maintenance medium, respectively. Finally, 100 µl of each dilution were placed in eight wells of a sterile polystyrene flat-bottomed plate with a preformed U373 monolayer Before the addition of the respective dilution, cells were washd twice with EMEM, and incubated for 3 hours 100 µl EMEM supplemented with trypsin. The cells were incubated at 37 °C in a CO2-atmosphere (5.0 % CO2 content). After six days of incubation, cultures were observed for cytopathic effects. The infectious dose (TCID50) was calculated according to the method of Spearman (1908) Brit. J. Psychol. 2, 227-242, and Kärber (1931) Arch. Exp. Path. Pharmak.; 162, 480-487.

### Controls

### (i) Virus control (VC)

Virus recovery was performed from non-treated infected U373-cells as described under 3.5. The mean virus titre was used as reference for calculation of the reduction factor.

### (ii) Irradiation without photosensitizer

In addition, virus recovery was performed from infected and radiated U373-cells without using of the Photolase sensitizer as

### (iii) Treatment with photosensitizer (without irradiation)

Another virus recovery was performed from infected U373-cells, treated with the Photolase sensitizer for 10 min under artificial laboratory light only (without radiation) as described in 3.5, whereby one Plate was wrapped up with aluminium foil immediately after addition of the dye (without radiation or light exposing) and stored and frozen in the dark until titration of residual virus.

### (iv) Cell culture control

Furthermore, a cell control (only addition of medium) was incorporated.

### Calculation of effectiveness

The virucidal effectiveness of the inventive treatment was evaluated by calculating the decrease in titre of the treated and radiated culture in comparison with the control titration of the culture without treatment (VC). The difference is given as reduction factor (RF).

Based on the EN 14476, the present photodynamic therapy system has a virucidal efficacy if the titre is reduced at least by 4 log10 steps after an irradiation treatment (EN 14476:2013+A2:2019: Chemical disinfectants and antiseptics - Quantitative suspension test for the evaluation of virucidal activity of chemicals disinfectants and antiseptics in human medicine test - Test method and requirements (phase 2, step 1)). This corresponds to an inactivation of ≥ 99.99 %

### Results

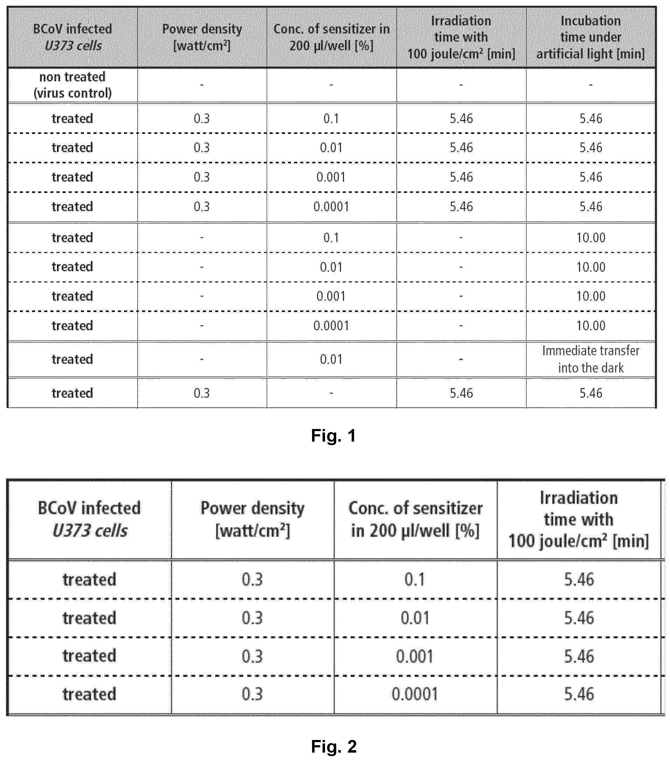

The effectiveness of the treatment of the infected cells according to the invention was determined after the irradiation treatment of three BCoV-infected U373 cell cultures of a 48-well plate (corresponds to three wells), respectively. Results of examination are shown in Fig. 2.

After adding the photolasesensitizer to the culture of BCoV-infected cells and irradiation with 810 nm at an energy density of 100 joule/cm² and under a constant power density of 0.3 watt/cm² no residual virus was found using 0.1% (w/v) to 0.001% (w/v) of the photosensitizer The reduction factor (RF) varied from 3.08 (with 0.1 % of the dye), to 4.08 (0.01 %),
and 5.08 (0.001 %). When using 0.0001 % of the photosensitizer only minimal residual virus could still be detected in one sample. However, the mean reduction factor was each ≥ 6.00 log₁₀ steps, since no cytotoxicity (CT) could be observed in these samples. This corresponded to a virus inactivation of ≥ 99.999 %.

In contrast, in tests of BCoV-infected cells that were treated with 0.01 % of photosensitizer (without radiation) and immediately wrapped in aluminium foil after addition of the sensitizer, and stored and frozen in the dark until titration of residual virus), substantial amounts of residual virus and no effectiveness could be detected (RF = 0.38 ± 0.64) (Fig. 2).

Test samples that were only irradiated and not treated with the sensitizer beforehand showed also no effectiveness (RF = 0.08 ± 0.64) (Fig. 2).

The present Example shows that the inventive treatment of cells infected with a high dose of bovine coronavirus leads to an effective virus inhibition using a composition containing the photosensitizer at concentrations of as low as 0.0001 % (w/v).

### Example 2: Inactivation of bovine coronavirus in infected cells in vitro using non-coherent LED light

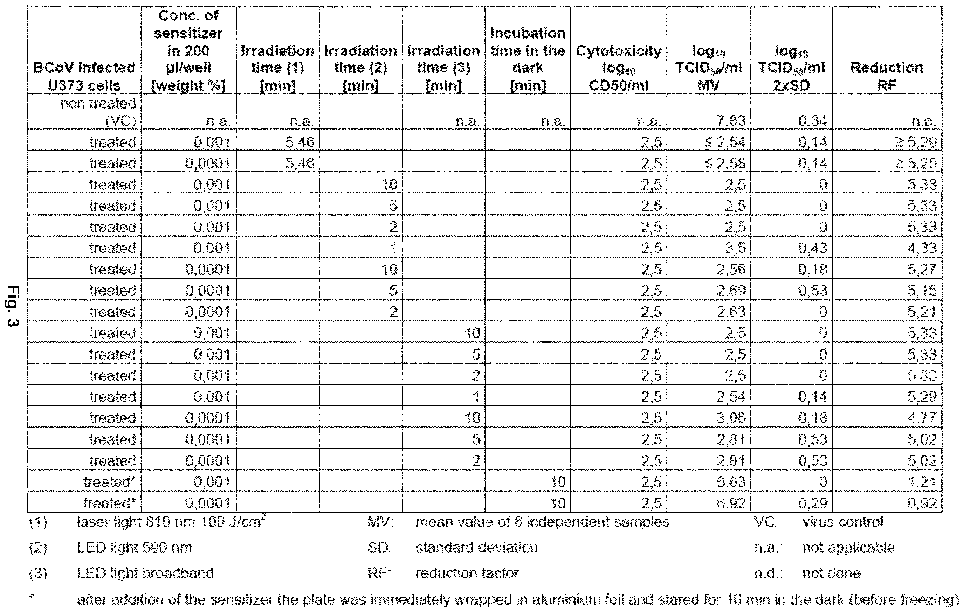

In a further set of experiments, the U737 cells were treated under the conditions as shown in the table of Fig. 3 using irradiation with either a monochromatic LED device at 590 nm or a broadband LED device (non-coherent light in each case). Light intensity was 10000 Lux in both cases.

Surprisingly, broadband LED light was successfully applied for photosensitizer activation leading to reduction of residual virus by 3 log₁₀ steps after 2 min of irradiation.

## Claims

1. A photosensitizer for use in the prevention and/or treatment of viral respiratory infections caused by a coronavirus comprising applying the photosensitizer intra-nasally and/or intra-orally and/or pharyngeally and irradiating at least a part of the respiratory tract of a subject from outside of the subject with light comprising or having a wavelength selected from 500 nm to 820 nm of the electromagnetic spectrum,
wherein the photosensitizer is a phenothiazine derivative having a structure of the following formula (I) 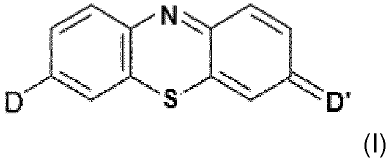
wherein
D and D' each are an electron donor group, and wherein the positions C-1, C-2, C-4, C-6, C-8 and/or C-9 of the phenothiazine ring system may be substituted by a substituent preferably selected from substituted or unsubstituted lower alkyl, preferably C₁₋₃-alkyl, particularly preferred methyl, or lower alkenyl, preferably C₂₋₃-alkenyl, amino, halo, and cyano. Preferably, D and D' are each independently a NRR' group with R and R' being preferably independently selected from H, substituted or unsubstituted lower alkyl, preferably C₁₋₃-alkyl, particularly preferred methyl, and lower alkenyl, preferably C₂₋₃-alkenyl.

2. The photosensitizer for use of claim 1 wherein the light is emitted by a light emitting diode (LED) device.

3. The photosensitizer for use of claim 2 wherein the LED device is a handheld LED device.

4. The photosensitizer for use of claim 2 wherein the LED device is a laser LED device, or a non-coherent light emitting LED device, preferably a broadband LED device.

5. The photosensitizer for use of claim 4 wherein the laser LED device is a handheld laser LED device.

6. The photosensitizer for use according to any one of the preceding claims wherein the at least part of the respiratory tract is irradiated one or more times for a period of from 2 to 20 min, preferably from 2 to 10 min, more preferably from 3 to 8 min.

7. The photosensitizer for use of claim 6 wherein the at least part of the respiratory tract is irradiated from outside of the subject with an energy density of 50 to 150 J/cm², preferably 80 to 120 J/cm², more preferably 90 to 110 J/cm², most preferably 100 J/cm².

8. The photosensitizer for use of according to any one of the preceding claims wherein the phenothiazine dye is selected from the group consisting of methylene blue, new methylene blue, toluidine blue and mixtures thereof.

9. The photosensitizer for use according to any one of the preceding claims wherein the phenothiazine derivative according to formula (I) is applied as a composition containing from 0.00001 (v/v) to 0.1 % (v/v), preferably from 0.0001 % (v/v) to 0.001 % (v/v), of said phenothiazine derivative.

10. The photosensitizer for use according to any one of the preceding claims wherein the light has a wavelength in the range of from 550 nm to 630 nm, preferably 570 to 610 nm, more preferably 585 nm to 595 nm, most preferred the light has a wavelength of 590 nm.

11. The photosensitizer for use according to any one of claims 1 to 9 wherein the light has a wavelength in the range of from 780 nm to 820 nm, preferably from about 800 nm to about 820 nm, most preferred the light has a wavelength of 810 nm.

12. The photosensitizer for use according to any one of the preceding claims wherein the coronavirus is selected from the group consisting of SARS-CoV, MERS-CoV, SARS-CoV 2, HCoV-HKU1, HCoV-OC43, HCoV-NL63, HCoV-229E and bovine coronaviruses (bCoVs).

13. The photosensitizer for use of claim 12 wherein the coronavirus is SARS-CoV 2.

14. The photosensitizer for use according to any one of the preceding claims wherein the photosensitizer is comprised in a pharmaceutical device, the pharmaceutical device comprising a nebulizer and a container containing at least one phenothiazine derivative as defined in claim 1, optionally in combination with at least one pharmaceutically acceptable excipient and/or diluent.

15. The photosensitizer for use of claim 14 wherein the phenothiazine derivative is present in sterilized water or in a sterilized aqueous solution.

16. The photosensitizer for use of claim 15 wherein the container contains the phenothiazine derivative at a concentration of from 0.00001 (v/v) to 0.1 % (v/v), preferably from 0.0001 % (v/v) to 0.001 % (v/v).

17. A medical kit comprising a pharmaceutical device as defined in any one of claims 14 to 16 and a handheld light source emitting light comprising or having a wavelength selected from 500 nm to 820 nm of the electromagnetic spectrum.

18. The kit of claim 17 wherein the light source is an LED device.

19. The kit of claim 18 wherein the light source is a broadband LED device, preferably emitting white light.

20. The kit of claim 19 wherein the LED device is a laser LED device.

21. The kit of claim 20 wherein the laser LED device emits light having a wavelength in the range of from 550 nm to 630 nm, preferably 570 to 610 nm, more preferably 585 nm to 595 nm, most preferred the light has a wavelength of 590 nm.

22. The kit of claim 20 wherein the laser LED device emits light having a wavelength in the range of from 780 nm to 820 nm, preferably from about 800 nm to about 820 nm, most preferred the light has a wavelength of 810 nm.

23. The kit of claim 18 wherein the LED device emits in-coherent monochromatic light having a wavelength in the range of from 550 nm to 630 nm, preferably 570 to 610 nm, more preferably 585 nm to 595 nm, most preferred the light has a wavelength of 590 nm.

24. The kit of claim 18 wherein the LED device emits in-coherent monochromatic light having a wavelength in the range of from 780 nm to 820 nm, preferably from about 800 nm to about 820 nm, most preferred the light has a wavelength of 810 nm.

## Patentansprüche

1. Photosensibilisator zur Verwendung in der Prävention und/oder Behandlung von viralen respiratorischen Infektionen, die durch einen Coronavirus hervorgerufen werden, umfassend das intra-nasale und/oder intra-orale und/oder pharyngale Verabreichen des Photosensibilisators und Bestrahlen mindestens eines Teils des respiratorischen Trakts einer Person von außerhalb der Person mit Licht, mit einer oder umfassend eine Wellenlänge, die aus 500 nm bis 820 nm des elektromagnetischen Spektrums ausgewählt ist,
wobei der Photosensibilisator ein Phenothiazin-Derivat mit einer Struktur der folgenden Formel (I) 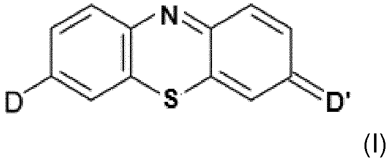
ist, wobei
D und D' jeweils eine Elektronendonorgruppe sind, und wobei die Positionen C-1, C2, C-4, C-8 und/oder C-9 des Phenothiazinringsystems mit einem Substituenten substituiert sein können, der vorzugsweise aus substituiertem oder nicht substituiertem Niederalkyl, vorzugsweise C₁₋₃-Alkyl, insbesondere bevorzugt Methyl, oder Niederalkenyl, vorzugsweise C₂₋₃-Alkenyl, Amino, Halo und Cyano, ausgewählt ist, wobei vorzugsweise D und D' jeweils unabhängig voneinander eine NRR'-Gruppe sind, wobei R und R' vorzugsweise unabhängig voneinander aus H, substituiertem oder nicht substituiertem Niederalkyl, vorzugsweise C₁₋₃-Alkyl, insbesondere bevorzugt Methyl, oder Niederalkenyl, vorzugsweise C₂₋₃-Alkenyl, ausgewählt sind.

2. Photosensibilisator zur Verwendung nach Anspruch 1, wobei das Licht durch eine Licht emittierende Dioden (LED)-Vorrichtung emittiert wird.

3. Photosensibilisator zur Verwendung nach Anspruch 2, wobei die LED-Vorrichtung eine Hand-LED-Vorrichtung ist.

4. Photosensibilisator zur Verwendung nach Anspruch 2, wobei die LED-Vorrichtung eine LED-Laservorrichtung oder eine inkohärentes Licht emittierende LED-Vorrichtung, vorzugsweise eine Breitband-LED-Vorrichtung ist.

5. Photosensibilisator zur Verwendung nach Anspruch 4, wobei LED-Laservorrichtung eine Hand-LED-Laservorrichtung ist.

6. Photosensibilisator zur Verwendung nach einem der vorhergehenden Ansprüche, wobei mindestens ein Teil des respiratorischen Trakts einmal oder mehrmals für eine Zeitspanne von 2 bis 20 min, vorzugsweise von 2 bis 10 min, mehr bevorzugt von 3 bis 8 min, bestrahlt wird.

7. Photosensibilisator zur Verwendung nach Anspruch 6, wobei der respiratorische Trakt von außerhalb der Person mit einer Energiedichte von 50 bis 150 J/cm², vorzugsweise von 50 bis 150 J/cm², mehr bevorzugt von 90 bis 110 J/cm², am meisten bevorzugt von 100 J/cm², bestrahlt wird.

8. Photosensibilisator zur Verwendung nach einem der vorhergehenden Ansprüche, wobei der Phenothiazin-Farbstoff aus der Gruppe, bestehend aus Methylenblau, Neumethylenblau, Toluidinblau und Gemischen davon, ausgewählt ist.

9. Photosensibilisator zur Verwendung nach einem der vorhergehenden Ansprüche, wobei das Phenothiazin-Derivat gemäß Formel (i) in einer Zusammensetzung verabreicht wird, die 0,00001 % (v/v) bis 0,1 % (v/v), vorzugsweise 0,0001 % (v/v) bis 0,001 % (v/v) des Phenothiazin-Derivats enthält.

10. Photosensibilisator zur Verwendung nach einem der vorhergehenden Ansprüche, wobei das Licht eine Wellenlänge im Bereich von 550 nm bis 630 nm, vorzugsweise 570 bis 610 nm, mehr bevorzugt 585 nm bis 595 nm, aufweist, wobei das Licht am meisten bevorzugt eine Wellenlänge von 590 nm aufweist.

11. Photosensibilisator zur Verwendung nach einem der Ansprüche 1 bis 9, wobei das Licht eine Wellenlänge im Bereich von 780 nm bis 820 nm, vorzugsweise von etwa 800 bis etwa 820 nm, aufweist, wobei das Licht am meisten bevorzugt eine Wellenlänge von 810 nm aufweist.

12. Photosensibilisator zur Verwendung nach einem der vorhergehenden Ansprüche, wobei das Coronavirus aus der Gruppe, bestehend aus SARS-CoV, MERS-CoV, SARS-CoV 2, HCoV-HKU1, HCoV-OC43, HCoV-NL63, HCoV229E und Rindercoronaviren (BCoV), ausgewählt ist.

13. Photosensibilisator zur Verwendung nach Anspruch 12, wobei das Coronavirus SARS-CoV 2 ist.

14. Photosensibilisator zur Verwendung nach einem der vorhergehenden Ansprüche, wobei der Photosensibilisator in einer pharmazeutischen Vorrichtung enthalten ist, die einen Vernebler und einen Behälter umfasst, der mindestens ein Phenothiazin-Derivat gemäß der Definition im Anspruch 1 ggf. in Kombination mit mindestens einem pharmazeutisch verträglichen Hilfsstoff und/oder Verdünnungsmittel enthält.

15. Photosensibilisator zur Verwendung nach Anspruch 14, wobei das Phenothiazin-Derivat in sterilisiertem Wasser oder in einer sterilisierten wässerigen Lösung vorliegt.

16. Photosensibilisator zur Verwendung nach Anspruch 15, wobei der Behälter das Phenothiazin-Derivat in einer Konzentration von 0,00001 % (v/v) bis 0,1 % (v/v), vorzugsweise 0,0001 % (v/v) bis 0,001 % (v/v), enthält.

17. Medizinisches Kit, das eine pharmazeutische Vorrichtung gemäß der Definition in einem der Ansprüche 14 bis 16 und eine Handlichtquelle umfasst, die Licht mit einer oder umfassend eine Wellenlänge emittiert, die aus 500 nm bis 820 nm des elektromagnetischen Spektrums ausgewählt ist.

18. Kit nach Anspruch 17, wobei die Lichtquelle eine LED-Vorrichtung ist.

19. Kit nach Anspruch 18, wobei die Lichtquelle eine Breitband-LED-Vorrichtung ist, die vorzugsweise weißes Licht emittiert.

20. Kit nach Anspruch 19, wobei die LED-Vorrichtung eine Laser-LED-Vorrichtung ist.

21. Kit nach Anspruch 20, wobei die Laser-LED-Vorrichtung Licht mit einer Wellenlänge im Bereich von 550 nm bis 630 nm, vorzugsweise von 570 bis 610 nm, mehr bevorzugt 585 nm bis 595 nm, emittiert, wobei das Licht am meisten bevorzugt eine Wellenlänge von 590 nm aufweist.

22. Kit nach Anspruch 20, wobei die Laser-LED-Vorrichtung Licht mit einer Wellenlänge im Bereich von 780 nm bis 820 nm, vorzugsweise von etwa 800 bis etwa 820 nm, emittiert, wobei das Licht am meisten bevorzugt eine Wellenlänge von 810 nm aufweist.

23. Kit nach Anspruch 18, wobei die LED-Vorrichtung inkohärentes monochromatisches Licht mit einer Wellenlänge im Bereich von 550 nm bis 630 nm, vorzugsweise von 570 bis 610 nm, mehr bevorzugt 585 nm bis 595 nm, emittiert, am meisten bevorzugt weist das Licht eine Wellenlänge von 590 nm auf.

24. Kit nach Anspruch 18, wobei die LED-Vorrichtung inkohärentes monochromatisches Licht mit einer Wellenlänge im Bereich von 780 nm bis 820 nm, vorzugsweise von etwa 800 bis etwa 820 nm, emittiert, wobei das Licht am meisten bevorzugt eine Wellenlänge von 810 nm aufweist.

## Revendications

1. Photosensibilisateur pour la mise on oeuvre dans la prévention et/ou le traitement des infections respiratoires virales causées par un coronavirus, comprenant l'application du photosensibilisateur par voie intra-nasale et/ou intra-orale et/ou pharyngée et l'irradiation d'au moins une partie des voies respiratoires d'un sujet depuis l'extérieur du sujet avec une lumière comprenant ou ayant une longueur d'onde choisie entre 500 nm et 820 nm du spectre électromagnétique,
dans lequel le photosensibilisateur est un dérivé de phénothiazine ayant une structure de la formule suivante (I) 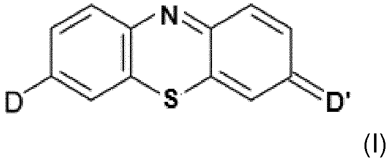
dans laquelle
D et D' sont chacun un groupe donneur d'électrons, et dans laquelle les positions C-1, C-2, C-4, C-6, C-8 et/ou C-9 du système cyclique de la phénothiazine peuvent être substituées par un substituant choisi de préférence parmi les alkyles inférieurs substitués ou non substitués, de préférence les alkyles en C1-3, en particulier le méthyle, ou les alcényles inférieurs, de préférence les alcényles en C2-3, l'amino, l'halo et le cyano. De préférence, D et D' sont chacun indépendamment un groupe NRR', R et R' étant de préférence indépendamment choisis parmi H, alkyle inférieur substitué ou non substitué, de préférence C1-3-alkyle, de préférence méthyle, et alcényle inférieur, de préférence C2-3-alkényle.

2. Le photosensibilisateur pour la mise on oeuvre selon la revendication 1, dans lequel la lumière est émise par une diode électroluminescente (DEL).

3. Le photosensibilisateur pour la mise on oeuvre selon la revendication 2, dans lequel le dispositif DEL est un dispositif DEL portatif.

4. Le photosensibilisateur pour la mise on oeuvre selon la revendication 2, dans lequel le dispositif DEL est un dispositif DEL laser ou un dispositif DEL émettant une lumière non cohérente, de préférence un dispositif DEL à large bande.

5. Photosensibilisateur pour la mise on oeuvre selon la revendication 4, dans lequel le dispositif laser LED est un dispositif laser LED portatif.

6. Le photosensibilisateur pour la mise on oeuvre selon l'une des revendications précédentes dans lequel la partie au moins des voies respiratoires est irradiée une ou plusieurs fois pendant une période allant de 2 à 20 min, de préférence de 2 à 10 min, plus préférentiellement de 3 à 8 min.

7. Le photosensibilisateur pour la mise on oeuvre selon la revendication 6, dans lequel la partie au moins des voies respiratoires est irradiée de l'extérieur du sujet avec une densité d'énergie de 50 à 150 J/cm², de préférence de 80 à 120 J/cm², de préférence de 90 à 110 J/cm², de préférence encore de 100 J/cm².

8. Photosensibilisateur pour la mise on oeuvre selon l'une quelconque des revendications précédentes, dans lequel le colorant phénothiazine est choisi dans le groupe constitué par le bleu de méthylène, le nouveau bleu de méthylène, le bleu de toluidine et leurs mélanges.

9. Le photosensibilisateur pour la mise on oeuvre selon l'une quelconque des revendications précédentes dans lequel le dérivé de phénothiazine selon la formule (I) est appliqué en tant que composition contenant de 0,00001 (v/v) à 0,1 % (v/v), de préférence de 0,0001 % (v/v) à 0,001 % (v/v), dudit dérivé de phénothiazine.

10. Le photosensibilisateur pour la mise on oeuvre selon l'une des revendications précédentes dans lequel la lumière a une longueur d'onde comprise entre 550 nm et 630 nm, de préférence entre 570 et 610 nm, plus préférentiellement entre 585 nm et 595 nm, de préférence encore entre 590 nm et 595 nm.

11. Le photosensibilisateur pour la mise on oeuvre selon l'une des revendications 1 à 9, dans lequel la lumière a une longueur d'onde comprise entre 780 nm et 820 nm, de préférence entre environ 800 nm et environ 820 nm, et de préférence encore une longueur d'onde de 810 nm.

12. Photosensibilisateur pour la mise on oeuvre selon l'une des revendications précédentes, dans lequel le coronavirus est choisi dans le groupe constitué par le SARS-CoV, le MERS-CoV, le SARS-CoV 2, le HCoV-HKU1, le HCoV-OC43, le HCoV-NL63, le HCoV-229E et les coronavirus bovins (bCoV).

13. Photosensibilisateur pour la mise on oeuvre selon la revendication 12, dans lequel le coronavirus est le SARS-CoV 2.

14. Le photosensibilisateur pour la mise on oeuvre selon l'une des revendications précédentes, dans lequel le photosensibilisateur est compris dans un dispositif pharmaceutique comprenant un nébuliseur et un récipient contenant au moins un dérivé de phénothiazine tel que défini dans la revendication 1, éventuellement en combinaison avec au moins un excipient et/ou un diluant pharmaceutiquement acceptable.

15. Le photosensibilisateur pour la mise on oeuvre selon la revendication 14 dans lequel le dérivé de phénothiazine est présent dans de l'eau stérilisée ou dans une solution aqueuse stérilisée.

16. Le photosensibilisateur pour la mise on oeuvre selon la revendication 15, dans lequel le récipient contient le dérivé de phénothiazine à une concentration de 0,00001 (v/v) à 0,1 % (v/v), de préférence de 0,0001 % (v/v) à 0,001 % (v/v).

17. Kit médical comprenant un dispositif pharmaceutique tel que défini dans l'une des revendications 14 à 16 et une source lumineuse portable émettant une lumière comprenant ou ayant une longueur d'onde sélectionnée entre 500 nm et 820 nm du spectre électromagnétique.

18. Le kit selon la revendication 17, dans lequel la source lumineuse est un dispositif LED.

19. Le kit selon la revendication 18, dans lequel la source lumineuse est un dispositif LED à large bande, émettant de préférence une lumière blanche.

20. Le kit selon la revendication 19 dans lequel le dispositif LED est un dispositif LED laser.

21. Le kit selon la revendication 20 dans lequel le dispositif laser LED émet une lumière ayant une longueur d'onde comprise entre 550 nm et 630 nm, de préférence entre 570 et 610 nm, plus préférentiellement entre 585 nm et 595 nm, la longueur d'onde la plus préférée étant de 590 nm.

22. Le kit selon la revendication 20, dans lequel le dispositif laser LED émet une lumière d'une longueur d'onde comprise entre 780 nm et 820 nm, de préférence entre 800 nm et 820 nm environ, et de préférence encore une lumière d'une longueur d'onde de 810 nm.

23. Le kit selon la revendication 18 dans lequel le dispositif LED émet une lumière monochromatique incohérente d'une longueur d'onde comprise entre 550 nm et 630 nm, de préférence entre 570 et 610 nm, plus préférentiellement entre 585 nm et 595 nm, la lumière la plus préférée ayant une longueur d'onde de 590 nm.

24. Le kit selon la revendication 18 dans lequel le dispositif LED émet une lumière monochromatique cohérente d'une longueur d'onde comprise entre 780 nm et 820 nm, de préférence entre environ 800 nm et environ 820 nm, la longueur d'onde la plus préférée étant de 810 nm.
